# EUROPÄISCHE PATENTANMELDUNG

(11) **EP 2 143 414 A2**
(43) Veröffentlichungstag der Anmeldung: **13.01.2010**
(21) Anmeldenummer: 09153077.4
(22) Anmeldetag: 18.02.2009
(51) Int. Cl.: A61K 8/23, A61K 8/41, A61K 8/44, A61Q 5/10

(54) **Oxidationsfärbemittel mit basischer Aminosäure, Ammoniumsulfat und Alkanolaminen**

(30) Priorität: 09.07.2008 DE 102008032208
(71) Anmelder: Henkel AG & Co. KGaA, 40589 Düsseldorf (DE)
(72) Erfinder: Goutsis, Konstantin, 41812, Erkelenz (DE); Reichert, Anja, 40629, Düsseldorf (DE)

(57) **Zusammenfassung**

Gegenstand der Erfindung ist ein Mittel zum Färben von keratinischen Fasern, dadurch gekennzeichnet, dass es in einem wässrigen Träger mindestens ein Oxidationsfarbstoffvorprodukt, mindestens eine basische Aminosäure, 0,3 bis 10 Gew.-% Ammoniumsulfat, bezogen auf das Gesamtgewicht der Anwendungsmischung, und mindestens ein Alkanolamin enthält. Die erfindungsgemäßen Mittel zeichnen sich durch gute Färbeeigenschaften bei gleichzeitigem Schutz und Pflege für das menschliche Haar aus.

## Beschreibung

Die Erfindung betrifft ein oxidatives Färbemittel für keratinhaltige Fasern, welches in einem wässrigen Träger mindestens ein Oxidationsfarbstoffvorprodukt sowie eine haarschützende und -pflegende Kombination aus einer basischen Aminosäure, Ammoniumsulfat und Alkanolaminen enthält. Weiterhin betrifft die Erfindung ein Verfahren mit verkürzter Einwirkzeit von solchen oxidativen Färbemitteln auf keratinhaltigen Fasern. Darüber hinaus betrifft die Erfindung die Verwendung des besagten Mittels zur Färbung von Haaren bei gleichzeitiger Pflege.

Die Veränderung von Form und Farbe der Haare stellt einen wichtigen Bereich der modernen Kosmetik dar. Dadurch kann das Erscheinungsbild der Haare sowohl aktuellen Modeströmungen als auch den individuellen Wünschen der einzelnen Person angepasst werden. Zur modischen Farbgestaltung von Frisuren oder zur Kaschierung von ergrautem oder gar weißem Haar mit modischen oder natürlichen Farbtönen greift der Verbraucher zu farbverändernden Mitteln. Diese Mittel sollen neben der gewünschten Färbeleistung möglichst minimale Schädigungen auf dem Haar hervorrufen und vorzugsweise sogar zusätzliche Pflegeeigenschaften besitzen.

Zur Bereitstellung farbverändernder kosmetischer Mittel, insbesondere für die Haut oder keratinhaltige Fasern wie beispielsweise menschliche Haare, kennt der Fachmann je nach Anforderungen an die Färbung diverse Färbesysteme.

Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich zwar durch hervorragende, lang anhaltende Färbeergebnisse aus. Für natürlich wirkende Färbungen muss aber üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet.

Als Entwicklerkomponenten werden üblicherweise primäre aromatische Amine mit einer weiteren, in para- oder ortho-Position befindlichen, freien oder substituierten Hydroxy- oder Aminogruppe, heterozyklische Hydrazone, Diaminopyrazolderivate sowie 2,4,5,6-Tetraaminopyrimidin und dessen Derivate eingesetzt. Als Kupplerkomponenten werden in der Regel m-Phenylendiaminderivate, Naphthole, Pyridinderivate, Resorcin und Resorcinderivate, Pyrazolone und m-Aminophenole verwendet.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Substrat aufziehen und keinen oxidativen Prozess zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel deutlich empfindlicher als die oxidativen Färbungen, so dass dann sehr viel schneller eine vielfach unerwünschte Nuancenverschiebung oder gar ein sichtbarer homogener Farbverlust eintritt.

Schließlich hat ein weiteres Färbeverfahren große Beachtung gefunden. Bei diesem Verfahren werden Vorstufen des natürlichen Haarfarbstoffes Melanin auf das Substrat, z. B. Haare, aufgebracht; diese bilden dann im Rahmen oxidativer Prozesse im Haar naturanaloge Farbstoffe aus. Bei, insbesondere mehrfacher, Anwendung von Mitteln mit 5,6-Dihydroxyindolin ist es möglich, Menschen mit ergrauten Haaren die natürliche Haarfarbe wiederzugeben. Die Ausfärbung kann dabei mit Luftsauerstoff als einzigem Oxidationsmittel erfolgen, so dass auf keine weiteren Oxidationsmittel zurückgegriffen werden muss. Bei Personen mit ursprünglich mittelblondem bis braunem Haar kann das Indolin als alleinige Farbstoffvorstufe eingesetzt werden. Für die Anwendung bei Personen mit ursprünglich roter und insbesondere dunkler bis schwarzer Haarfarbe können dagegen befriedigende Ergebnisse häufig nur durch Mitverwendung weiterer Farbstoffkomponenten, insbesondere spezieller Oxidationsfarbstoffvorprodukte, erzielt werden.

Sollen Substrate aufgehellt oder gar gebleicht werden, werden die das Substrat färbenden Farbstoffe meist oxidativ unter Einsatz von entsprechenden Oxidationsmitteln, wie beispielsweise Wasserstoffperoxid, entfärbt.

Insbesondere oxidative Haarfärbemittel sind trotz ihrer vorteilhaften Färbeeigenschaften für den Anwender mit Nachteilen behaftet. Einerseits führt der Einsatz der Oxidationsmittel zur Ausfärbung respektive Entwicklung der eigentlichen Färbung zu Schädigungen in der Haarstruktur und auf der Haaroberfläche. Das Haar wird brüchig, seine Elastizität lässt nach und die Kämmbarkeit nimmt ab. Diese Schädigung nimmt mit der Anwendungsdauer zu. Handelsübliche oxidative Färbemittel müssen meist über einen Zeitraum von 30 Minuten und länger auf die Haarfaser einwirken. Sollen graue oder weiße Haare kaschiert werden, so muss für eine bessere Grauabdeckung die Einwirkzeit eines oxidativen Färbemittels gegebenenfalls noch weiter erhöht werden. Die Erhöhung der Einwirkzeit führt zu einer gesteigerten Beeinträchtigung der Haarstruktur. Es ist auch aus Gründen des Anwendungskomforts nahe liegend, dass bei den Benutzern solcher Haarfärbemittel ein Bedürfnis besteht, diese Einwirkungszeit zu verringern.

Andererseits benötigen oxidative Färbemittel in der Regel einen basischen pH-Wert zur Ausfärbung, insbesondere zwischen pH 9,0 und pH 10,5. Diese pH-Werte sind notwendig, um eine Öffnung der äußeren Schuppenschicht (Cuticula) zu gewährleisten und eine Penetration der aktiven Spezies (Farbstoffvorprodukte und/oder Wasserstoffperoxid) in das Haar zu ermöglichen. Das basische Milieu stellt jedoch einen weiteren Grund der Schädigung für das Haar und dessen Struktur dar, der ebenfalls mit gesteigerter Anwendungszeit an Bedeutung gewinnt. Die Spreizung der äußeren Schuppenschicht führt außerdem zu einem unangenehmen Oberflächenempfinden der Haare und damit zu einer verschlechterten Kämmbarkeit im Nass- und Trockenzustand. Dadurch besteht für den Verbraucher eine gesteigerte Notwendigkeit, zusätzliche Nachbehandlungsmittel wie Konditioniermittel einsetzen.

Schließlich wird der basische pH-Wert häufig mit Ammoniak als Alkalisierungsmittel eingestellt, da ammoniakhaltige Färbemittel zusätzliche Vorteile hinsichtlich der Färbeleistung besitzen. Für den Anwender besitzt ein solches Färbemittel jedoch den Nachteil, dass es durch Ammoniak neben zusätzlichen Schädigungen des Haares zu Reizungen von Augen oder Kopfhaut kommen kann, wodurch Sensibilisierungen oder gar allergische Reaktionen hervorgerufen werden können. Darüber hinaus besitzen solche Färbemittel einen intensiven, unangenehmen Geruch, der schlimmstenfalls auch zu Reizungen der Nasenschleimhaut führen kann. Ausserdem kann auch die Produktion und Lagerung von Ammoniak-haltigen Färbemitteln mit Problemen hinsichtlich Handhabbarkeit und Stabilität verbunden sein.

Aufgabe der vorliegenden Erfindung ist es daher, die oben genannten Nachteile oxidativer Haarfärbemittel herabzusenken. Die Färbemittel sollen das Haar schützen und damit eine verringerte Schädigung des Haares bewirken. Insbesondere Schutz vor oxidativen Schädigungen der Haarstruktur und der Haaroberfläche soll durch die Haarfärbemittel erzielt werden. Besonders wünschenswert sind pflegende Eigenschaften der Mittel, so dass der Anwender auf den Einsatz zusätzlicher Konditioniermittel verzichten kann. Die Färbemittel sollen weiterhin durch eine Verkürzung der Anwendungsdauer bei einem Erhalt der Färbeleistung zulassen, wodurch eine weitere Reduktion der Schädigung ermöglicht wird. Außerdem ist eine Verkürzung der Anwendungszeit für den Verbraucher als Erleichterung in der Anwendung erstrebenswert. Schließlich ist es wünschenswert, oxidative Haarfärbemittel bereitzustellen, die weitgehend oder vollständig auf den Zusatz von Ammoniak verzichten.

In nicht vorhersehbarer Weise konnte nun gefunden werden, dass der Einsatz von oxidativen Haarfärbemitteln, die neben Oxidationsfarbstoffvorprodukten eine Kombination aus einer basischen Aminosäure, einer signifikanten Menge an Ammoniumsulfat und einem Alkanolamin enthalten, eine intensive Färbung von keratinischen Fasern, insbesondere menschlicher Haare, ermöglicht. Die erfindungsgemäßen Färbemittel können weiterhin auf den Zusatz von Ammoniak verzichten. Bedingt durch die Schutzwirkung bei Anwendung des erfindungsgemäßen Mittels kann die Haarschädigung hierdurch minimiert werden oder sogar eine Haarpflege erzielt werden. Auch eine Verkürzung der Einwirkzeit unter Erzielung eines dem Stand der Technik entsprechenden Färbeeffekts ist auf diese Weise möglich.

Ein erster Gegenstand der Erfindung ist daher ein Mittel zum Färben keratinischer Fasern, insbesondere menschlicher Haare, enthaltend in einem kosmetischen Träger mindestens ein Oxidationsfarbstoffvorprodukt, **dadurch gekennzeichnet, dass** das Färbemittel zusätzlich
a) mindestens eine basische Aminosäure,
b) 0,3 bis 10 Gew.-% Ammoniumsulfat, bezogen auf das Gesamtgewicht der Anwendungsmischung,
c) und mindestens ein Alkanolamin enthält.

Unter keratinischen Fasern oder auch Keratinfasern sind dabei Pelze, Wolle, Federn und insbesondere menschliche Haare zu verstehen. Obwohl die erfindungsgemäßen Mittel in erster Linie zum Färben von Keratinfasern geeignet sind, steht prinzipiell einer Verwendung auch auf anderen Gebieten nichts entgegen.

Die erfindungsgemäßen Mittel enthalten die Wirkstoffe in einem kosmetischen Träger. Dieser kosmetische Träger ist im Sinne der Erfindung ist wässrig, alkoholisch oder wässrig-alkoholisch. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind.

Unter wässrig-alkoholischen Trägern sind im Sinne der vorliegenden Erfindung wasserhaltige Zusammensetzungen, enthaltend 3 bis 70 Gew.-% eines C₁-C₄-Alkohols, bezogen auf das Gesamtgewicht der Anwendungsmischung, insbesondere Ethanol bzw. Isopropanol, zu verstehen. Die erfindungsgemäßen Mittel können zusätzlich weitere organische Lösungsmittel, wie beispielsweise 4-Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten. Bevorzugt sind dabei alle wasserlöslichen organischen Lösungsmittel.

Ein wässriger Träger enthält im Sinne der Erfindung mindestens 30 Gew.-%, insbesondere mindestens 50 Gew.-% Wasser, bezogen auf das Gesamtgewicht der Anwendungsmischung.

Als ersten wesentlichen Inhaltsstoff enthält das erfindungsgemäße Mittel eine basische Aminosäure. Als basische Aminosäure im Sinne der Erfindung gilt eine organische Verbindung, die in ihrer Struktur mindestens eine protonierbare Aminogruppe und mindestens eine -COOH- oder eine -SO₃H-Gruppe, deren 2,5-%ige Lösung in Wasser einen pH-Wert von größer 9,0 aufweist.

Bevorzugte basische Aminosäuren sind Aminocarbonsäuren, insbesondere α-Aminocarbonsäuren und ω-Aminocarbonsäuren. Unter den α-Aminocarbonsäuren sind wiederum Lysin, Ornithin und insbesondere Arginin besonders bevorzugt.

Die basischen Aminosäuren können den erfindungsgemäßen Mitteln bevorzugt in freier Form zugegeben werden. In einer Reihe von Fällen ist es jedoch auch vorteilhaft, die Aminosäuren in Salzform einzusetzen. Bevorzugte Salze sind dann die Verbindungen mit Halogenwasserstoffsäuren oder Schwefelsäure, insbesondere die Hydrochloride, die Hydrobromide und die Sulfate.

Weiterhin können die basischen Aminosäuren auch in Form von Oligopeptiden und Proteinhydrolysaten eingesetzt werden, wenn sichergestellt ist, dass die erforderlichen Mengen der erfindungsgemäß eingesetzten Aminosäuren darin enthalten sind. In diesem Zusammenhang wird auf die Offenbarung der DE-OS 22 15 303 verwiesen, auf die ausdrücklich Bezug genommen wird.

Eine erfindungsgemäß besonders bevorzugte basische Aminosäure ist Arginin, insbesondere in freier Form, aber auch als Hydrochlorid eingesetzt.

Die basische Aminosäure ist in den erfindungsgemäßen Oxidationsfärbemitteln bevorzugt in Mengen von 0,01 bis 10 Gew.-%, insbesondere von 0,05 bis 5 Gew.-%, bezogen auf das Gesamtgewicht der Anwendungsmischung, enthalten.

Als zweiter wesentlicher Inhaltsstoff des erfindungsgemäßen Mittels sind mindestens 0,3 bis 10 Gew.-% Ammoniumsulfat, bezogen auf das Gesamtgewicht der Anwendungsmischung, enthalten.

In den Untersuchungen zu dieser Erfindung hat sich herausgestellt, dass Ammoniumsulfat in den erfindungsgemäßen Mitteln teilweise oder vollständig in ionischer Form vorliegt. Je nach pH-Wert der Mittel befindet sich das kationische Ammonium-lon mehr oder weniger ausgeprägt im Gleichgewicht mit seiner Neutralform, dem Ammoniak. Das in den erfindungsgemäßen Mengen eingesetzte Ammoniumsulfat dient dabei als Ammonium-Ionen freisetzende Verbindung. Ammonium-Ionen bzw. Ammoniak selbst sind für eine waschbeständige, lang anhaltende Färbung mit guter Grauabdeckung besonders vorteilhaft. Insbesondere hinsichtlich der Ergebnisse zu Farbintensität, Waschechtheit und Grauabdeckungsvermögen sind Ammoniak-haltige Färbemittel Färbemitteln ohne Ammoniak/Ammonium-Ionen deutlich überlegen. Es hat sich herausgestellt, dass die erfindungsgemäßen Färbemittel mit Ammoniumsulfat eine ebenso hohe Färbeleistung besitzen wie Mittel, denen Ammoniak selbst zugesetzt wurde, jedoch nicht deren Nachteile aufweisen.

Erfindungsgemäß bevorzugte Mittel sind **dadurch gekennzeichnet, dass** Ammoniumsulfat zu 0,5 bis 5 Gew.-%, insbesondere zu 0,8 bis 3 Gew.-% und ganz besonders bevorzugt zu 0,8 bis 2 Gew.-%, jeweils bezogen auf das Gesamtgewicht der Anwendungsmischung, enthalten ist.

Ein dritter wesentlicher Inhaltsstoff der erfindungsgemäßen Mittel ist ein Alkanolamin. Die als erfindungsgemäßes Alkalisierungsmittel einsetzbaren Alkanolamine werden bevorzugt ausgewählt aus primären, sekundären oder tertiären Aminen mit einem C₂-C₆-Alkylgrundkörper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin), 3-Amino-propan-1-ol, 4-Aminobutan-1-ol, 5-Aminopentan-1-ol, 1-Aminopropan-2-ol (Monoisopropanolamin), 1-Aminobutan-2-ol, 1-Aminopentan-2-ol, 1-Aminopentan-3-ol, 1-Aminopentan-4-ol, 2-Amino-2-methyl-propanol, 2-Amino-2-methylbutanol, 3-Amino-2-methylpropan-1-ol, 1-Amino-2-methylpropan-2-ol, 3-Aminopropan-1,2-diol, 2-Amino-2-methylpropan-1,3-diol, 2-Amino-2-ethyl-1,3-propandiol, N,N-Dimethyl-ethanolamin, Methylglucamin, Triethanolamin, Diethanolamin und Triisopropanolamin.

Erfindungsgemäß besonders bevorzugte Alkanolamine werden ausgewählt aus der Gruppe, gebildet aus Monoethanolamin, Monoisopropanolamin, 2-Amino-2-methylpropanol, 2-Amino-2-methyl-1,3-propandiol, 2-Amino-2-ethyl-1,3-propandiol, 2-Amino-2-methylbutanol, 2-Aminobutanol, N,N-Dimethyl-ethanolamin, Methylglucamin, Triethanolamin, Diethanolamin und Triisopropanolamin.

Erfindungsgemäß ganz besonders bevorzugte Alkanolamine werden ausgewählt aus der Gruppe 2-Aminoethan-1-ol (Monoethanolamin), 2-Amino-2-methylpropan-1-ol und 2-Amino-2-methyl-propan-1,3-diol. Ein erfindungsgemäß insbesondere bevorzugtes Alkanolamin ist Monoethanolamin.

Es ist erfindungsgemäß besonders vorteilhaft, wenn das Mittel ammoniakarm, bevorzugt ammoniakfrei ist. Die Begriffe "ammoniakarm" bzw. "ammoniakfrei" im Sinne der Erfindung beziehen sich dabei auf die dem Mittel zugesetzte Ammoniakmenge, wobei der Ammoniakzusatz sowohl als wässrige, alkoholische, wässrig-alkoholische oder anderweitige Lösung als auch durch Ammoniakgaseinleitung oder durch Zusatz von verflüssigtem Ammoniak erfolgen kann. Die Begriffe "ammoniakfrei" bzw. "ammoniakarm" im Sinne der Erfindung beziehen sich insbesondere nicht auf im erfindungsgemäßen Mittel freigesetzten Ammoniak, der durch Deprotonierung von als Salz zugesetzten Ammonium-Kationen entsteht.

Bevorzugte, ammoniakarme Mittel enthalten weniger als 0,5 Gew.-%, insbesondere weniger als 0,1 Gew.-% und ganz besonders bevorzugt weniger als 0,05 Gew.-% zugesetzten Ammoniak, jeweils bezogen auf das Gesamtgewicht der Anwendungszubereitung. Ammoniakfrei im Sinne der Erfindung sind solche Mittel, denen kein Ammoniak durch eine der oben beschriebenen Methoden zugesetzt wurde. Solche Mittel sind ganz besonders bevorzugt.

Schließlich enthalten die erfindungsgemäßen Mittel mindestens ein Oxidationsfarbstoffvorprodukt. Die erfindungsgemäßen Mittel zum Färben keratinischer Fasern sind demnach **dadurch gekennzeichnet, dass** sie mindestens ein Oxidationsfarbstoffvorprodukt vom Entwicklertyp und/oder Kupplertyp enthalten. Vorzugsweise enthalten die erfindungsgemäßen Färbemittel mindestens ein Oxidationsfarbstoffvorprodukt vom Entwicklertyp und mindestens ein Oxidationsfarbstoffvorprodukt vom Kupplertyp.

Die Oxidationsfarbstoffvorprodukte werden bevorzugt in einer Menge von 0,005 bis 20 Gew.-%, bevorzugt von 0,05 bis 5 Gew.-% und besonders bevorzugt von 0,1 bis 5 Gew.-%, jeweils bezogen auf das anwendungsbereite Oxidationsfärbemittel, verwendet.

Die Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride und Hydrobromide oder der Sulfate einzusetzen.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1 : 0,5 bis 1 : 2 enthalten sein können.

Nachfolgend werden erfindungsgemäße Entwicklerkomponenten eingehender vorgestellt.

Bevorzugte p-Phenylendiamine werden ausgewählt aus einer oder mehrerer Verbindungen der Gruppe, die gebildet wird, aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(2-hydroxyethyl)-p-phenylendiamin, 4-N,N-Bis-(2-hydroxyethyl)amino-2-methylanilin, 4-N,N-Bis-(2-hydroxyethyl)-amino-2-chloranilin, 2-(2-Hydroxyethyl)-p-phenylendiamin, 2-(1,2-Dihydroxyethyl)-p-phenylendiamin, 2-Fluor-p-phenylendiamin, 2-Isopropyl-p-phenylendiamin, N-(2-Hydroxypropyl)-p-phenylendiamin, 2-Hydroxymethyl-p-phenylendiamin, N,N-Dimethyl-3-methyl-p-phenylendiamin, N-Ethyl-N-2-hydroxyethyl-p-phenylendiamin, N-(2,3-Dihydroxypropyl)-p-phenylendiamin, N-(4'-Aminophenyl)-p-phenylendiamin, N-Phenyl-p-phenylendiamin, 2-(2-Hydroxyethyloxy)-p-phenylendiamin, 2-Methoxymethyl-p-phenylendiamin, 2-(2-Acetylaminoethyloxy)-p-phenylendiamin, N-(2-Methoxyethyl)-p-phenylendiamin, N-(4-Amino-3-methylphenyl)-N-[3-(1H-imidazol-1-yl)propyl]-amin, 5,8-Diaminobenzo-1,4-dioxan sowie ihren physiologisch verträglichen Salzen. Erfindungsgemäß ganz besonders bevorzugte p-Phenylendiaminderivate sind ausgewählt aus mindestens einer Verbindung der Gruppe p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, N-(4-Amino-3-methylphenyl)-N-[3-(1H-imidazol-1-yl)propyl]amin, 2-Methoxymethyl-p-phenylendiamin sowie den physiologisch verträglichen Salzen dieser Verbindungen.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind. Bevorzugte zweikernige Entwicklerkomponenten werden insbesondere aus mindestens einer der folgenden Verbindungen ausgewählt: N,N'-Bis-(2-hydroxyethyl)-N,N'-bis-(4'-aminophenyl)-1,3-diamino-propan-2-ol, N,N'-Bis-(2-hydroxyethyl)-N,N'-bis-(4'-aminophenyl)-ethylendiamin, N,N'-Bis-(4'-aminophenyl)-tetramethylendiamin, N,N'-Bis-(2-hydroxyethyl)-N,N'-bis-(4'-aminophenyl)-tetramethylendiamin, N,N'-Bis-(4-(methylamino)phenyl)-tetramethylendiamin, N,N'-Diethyl-N,N'-bis-(4'-amino-3'-methylphenyl)-ethylendiamin, Bis-(2-hydroxy-5-aminophenyl)-methan, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan sowie ihre physiologisch verträglichen Salze. Ganz besonders bevorzugte zweikernige Entwicklerkomponenten werden ausgewählt unter N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)propan-2-ol, N,N'-Bis-(4-aminophenyl)-1,4-diaza-cycloheptan, 1,10-Bis-(2,5-diaminophenyl)-1,4,7,10-tetraoxadecan oder eines der physiologisch verträglichen Salze dieser Verbindungen.

Weiterhin kann es erfindungsgemäß bevorzugt sein, als Entwicklerkomponente ein p-Aminophenolderivat oder eines seiner physiologisch verträglichen Salze einzusetzen. Bevorzugte p-Aminophenole sind insbesondere p-Aminophenol, N-Methyl-p-aminophenol, 4-Amino-3-methylphenol, 4-Amino-3-fluorphenol, 2-Hydroxymethylamino-4-aminophenol, 4-Amino-3-hydroxy-methylphenol, 4-Amino-2-(2-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(2-hydroxyethyl-aminomethyl)-phenol, 4-Amino-2-(1,2-dihydroxyethyl)-phenol, 4-Amino-2-fluorphenol, 4-Amino-2-chlorphenol, 4-Amino-2,6-dichlorphenol, 4-Amino-2-(diethyl-aminomethyl)phenol sowie ihre physiologisch verträglichen Salze. Ganz besonders bevorzugte Verbindungen sind p-Aminophenol, 4-Amino-3-methylphenol, 4-Amino-2-aminomethylphenol, 4-Amino-2-(1,2-dihydroxyethyl)-phenol und 4-Amino-2-(diethylaminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise aus Pyrimidinderivaten, Pyrazolderivaten, Pyrazolopyrimidin-Derivaten bzw. ihren physiologisch verträglichen Salzen.

Bevorzugte Pyrimidin-Derivate sind insbesondere die Verbindungen 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere die Verbindungen, die ausgewählt werden unter 4,5-Diamino-1-methylpyrazol, 4,5-Diamino-1-(2-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert-butyl-1-methylpyrazol, 4,5-Diamino-1-tert.-butyl-3-methylpyrazol, 4,5-Diamino-1-(2-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2-aminoethyl)-amino-1,3-dimethylpyrazol, sowie deren physiologisch verträglichen Salze.

Unter den Pyrazolo[1,5-a]pyrimidinen sind bevozugt: Pyrazolo[1,5-a]pyrimidin-3,7-diamin; 2,5-Dimethyl-pyrazolo[1,5-a]pyrimidin-3,7-diamin; Pyrazolo[1,5-a]pyrimidin-3,5-diamin; 2,7-Dimethyl-pyrazolo[1,5-a]pyrimidin-3,5-diamin; 3-Aminopyrazolo[1,5-a]pyrimidin-7-ol; 3-Aminopyrazolo[1,5-a]pyrimidin-5-ol; 2-(3-Aminopyrazolo[1,5-a]pyrimidin-7-ylamino)ethanol; 2-(7-Aminopyrazolo[1,5-a]pyrimidin-3-ylamino)ethanol; 2-[(3-Aminopyrazolo[1,5-a]pyrimidin-7-yl)-(2-hydroxy-ethyl)amino]-ethanol; 2-[(7-Aminopyrazolo[1,5-a]pyrimidin-3-yl)-(2-hydroxy-ethyl)-amino]-ethanol; 5,6-Dimethyl-pyrazolo[1,5-a]pyrimidin-3,7-diamin; 2,6-Dimethylpyrazolo[1,5-a]pyrimidin-3,7-diamin; 3-Amino-7-dimethylamino-2,5-dimethylpyrazolo[1,5-a]pyrimidin; sowie ihre physiologisch verträglichen Salze und ihre tautomeren Formen, wenn ein tautomeres Gleichgewicht vorhanden ist.

Ganz besonders bevorzugte Entwicklerkomponenten werden ausgewählt, aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diaminopropan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxa-decan, p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)-phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, sowie den physiologisch verträglichen Salzen dieser Verbindungen.

Die Entwicklerkomponenten werden bevorzugt in einer Menge von 0,005 bis 20 Gew.-%, vorzugsweise 0,1 bis 5 Gew.-%, jeweils bezogen auf das anwendungsbereite Oxidationsfärbemittel, verwendet.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Kupplerkomponente zusätzlich mindestens eine Entwicklerkomponente zum Einsatz kommt.

Kupplerkomponenten im Sinne der Erfindung erlauben mindestens eine Substitution eines chemischen Restes des Kupplers durch die oxidierte Form der Entwicklerkomponente. Dabei bildet sich eine kovalente Bindung zwischen Kuppler- und Entwicklerkomponente aus. Kuppler sind bevorzugt zyklische Verbindungen, die am Zyklus mindestens zwei Gruppen tragen, ausgewählt aus (i) gegebenenfalls substituierten Aminogruppen und/oder (ii) Hydroxylgruppen. Wenn die zyklische Verbindung ein Sechsring (bevorzugt aromatisch) ist, so befinden sich die besagten Gruppen bevorzugt in ortho-Position oder meta-Position zueinander.

Erfindungsgemäße Kupplerkomponenten werden bevorzugt als mindestens eine Verbindung aus einer der folgenden Klassen ausgewählt:
m-Aminophenol und/oder dessen Derivate, m-Diaminobenzol und/oder dessen Derivate, o-Diaminobenzol und/oder dessen Derivate, o-Aminophenolderivate, wie beispielsweise o-Aminophenol, Naphthalinderivate mit mindestens einer Hydroxygruppe, Di- beziehungsweise Trihydroxybenzol und/oder deren Derivate, Pyridinderivate, Pyrimidinderivate, Monohydroxyindol-Derivate und/oder Monoaminoindol-Derivate, Monohydroxyindolin-Derivate und/oder Monoaminoindolin-Derivate, Pyrazolonderivate, Morpholinderivate, Chinoxalinderivate sowie Gemische aus zwei oder mehrer Verbindungen aus einer oder mehrerer dieser Klassen sind im Rahmen dieser Ausführungsform ebenso erfindungsgemäß.

Bevorzugte m-Aminophenol-Kupplerkomponenten werden ausgewählt aus mindestens einer Verbindung aus der Gruppe, die gebildet wird aus m-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)benzol, 3-Ethylamino-4-methylphenol, 2,4-Dichlor-3-aminophenol und den physiologisch verträglichen Salzen aller vorstehend genannten Verbindungen.

Bevorzugte m-Diaminobenzol-Kupplerkomponenten werden ausgewählt aus mindestens einer Verbindung aus der Gruppe, die gebildet wird aus m-Phenylendiamin, 2-(2,4-Diaminophenoxy)-ethanol, 1,3-Bis(2,4-diaminophenoxy)propan, 1-Methoxy-2-amino-4-(2'-hydroxyethylamino)-benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}-amino)ethanol, 2-[3-Morpholin-4-ylphenyl)amino]ethanol, 3-Amino-4-(2-methoxyethoxy)-5-methyl-phenylamin, 1-Amino-3-bis-(2'-hydroxyethyl)-aminobenzol und den physiologisch verträglichen Salzen aller vorstehend genannten Verbindungen.

Bevorzugte o-Diaminobenzol-Kupplerkomponenten werden ausgewählt aus mindestens einer Verbindung aus der Gruppe, die gebildet wird aus 3,4-Diaminobenzoesäure und 2,3-Diamino-1-methylbenzol und den physiologisch verträglichen Salzen aller vorstehend genannten Verbindungen.

Bevorzugte Di- beziehungsweise Trihydroxybenzole und deren Derivate werden ausgewählt aus mindestens einer Verbindung der Gruppe, die gebildet wird aus Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol.

Bevorzugte Pyridinderivate werden ausgewählt aus mindestens einer Verbindung der Gruppe, die gebildet wird aus 2,6-Dihydroxypyridin, 2-Amino-3-hydroxypyridin, 2-Amino-5-chlor-3-hydroxy-pyridin, 3-Amino-2-methylamino-6-methoxypyridin, 2,6-Dihydroxy-3,4-dimethylpyridin, 2,6-Di-hydroxy-4-methylpyridin, 2,6-Diaminopyridin, 2,3-Diamino-6-methoxypyridin, 3,5-Diamino-2,6-di-methoxypyridin, 3,4-Diaminopyridin, 2-(2-Methoxyethyl)amino-3-amino-6-methoxypyridin, 2-(4'-Methoxyphenyl)amino-3-aminopyridin, und den physiologisch verträglichen Salzen der vorgenannten Verbindungen.

Bevorzugte Naphthalinderivate mit mindestens einer Hydroxygruppe werden ausgewählt aus mindestens einer Verbindung der Gruppe, die gebildet wird aus 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin.

Bevorzugte Indolderivate werden ausgewählt aus mindestens einer Verbindung der Gruppe, die gebildet wird aus 4-Hydroxyindol, 6-Hydroxyindol und 7-Hydroxyindol und den physiologisch verträglichen Salzen der vorgenannten Verbindungen.

Besonders bevorzugte Indolinderivate werden ausgewählt aus mindestens einer Verbindung der Gruppe, die gebildet wird aus 4-Hydroxyindolin, 6-Hydroxyindolin und 7-Hydroxyindolin und den physiologisch verträglichen Salzen der vorgenannten Verbindungen.

Bevorzugte Pyrimidinderivate werden ausgewählt aus mindestens einer Verbindung der Gruppe, die gebildet wird aus 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und den physiologisch verträglichen Salzen der vorgenannten Verbindungen.

Erfindungsgemäß besonders bevorzugte Kupplerkomponenten werden ausgewählt unter 3-Aminophenol, 5-Amino-2-methylphenol, 3-Amino-2-chlor-6-methylphenol, 2-Hydroxy-4-amino-phenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)-amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)-aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Di-hydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder den physiologisch verträglichen Salzen der vorgenannten Verbindungen.

Die Kupplerkomponenten werden bevorzugt in einer Menge von 0,005 bis 20 Gew.-%, vorzugsweise 0,1 bis 5 Gew.-%, jeweils bezogen auf das anwendungsbereite Oxidationsfärbemittel, verwendet.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1:0,5 bis 1:3, insbesondere 1:1 bis 1:2, stehen können.

Weiterhin können die erfindungsgemäßen Mittel mindestens einen direktziehenden Farbstoff enthalten. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.

Die direktziehenden Farbstoffe werden jeweils bevorzugt in einer Menge von 0,001 bis 20 Gew.-%, bezogen auf die gesamte Anwendungszubereitung, eingesetzt. Die Gesamtmenge an direktziehenden Farbstoffen beträgt vorzugsweise höchstens 20 Gew.-%.

Direktziehende Farbstoffe können in anionische, kationische und nichtionische direktziehende Farbstoffe unterteilt werden.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1 und Acid Black 52 bekannten Verbindungen.

Bevorzugte kationische direktziehende Farbstoffe sind dabei
kationische Triphenylmethanfarbstoffe, wie beispielsweise Basic Blue 7, Basic Blue 26, Basic Violet 2 und Basic Violet 14, aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie direktziehende Farbstoffe, die einen Heterocyclus enthalten, der mindestens ein quaternäres Stickstoffatom aufweist, wie sie beispielsweise in der EP-A2-998 908 genannt werden.

Die Verbindungen, die unter den Bezeichnungen Basic Yellow 87, Basic Orange 31 und Basic Red 51 bekannt sind, sind ganz besonders bevorzugte kationische direktziehende Farbstoffe.

Die kationischen direktziehenden Farbstoffe, die unter dem Warenzeichen Arianor^{®} vertrieben werden, sind erfindungsgemäß ebenfalls ganz besonders bevorzugte kationische direktziehende Farbstoffe.

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Es ist nicht erforderlich, dass die direktziehenden Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Weiterhin können als direktziehende Farbstoffe auch in der Natur vorkommende Farbstoffe eingesetzt werden, wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu und Alkannawurzel enthalten sind.

Als Farbstoffvorstufen naturanaloger Farbstoffe werden bevorzugt solche Indole und Indoline eingesetzt, die mindestens zwei Gruppen ausgewählt aus Hydroxy- und/oder oder Aminogruppen, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer weiteren Ausführungsform enthalten die Färbemittel mindestens ein Indol- und/oder Indolinderivat. Erfindungsgemäße Zusammensetzungen, die Vorstufen naturanaloger Farbstoffe enthalten, werden bevorzugt als luftoxidative Färbemittel verwendet. In dieser Ausführungsform werden die besagten Zusammensetzungen folglich nicht mit einem zusätzlichen Oxidationsmittel versetzt.

Die Farbstoffvorstufen naturanaloger Farbstoffe werden jeweils bevorzugt in einer Menge von 0,001 bis 5 Gew.-%, bezogen auf die gesamte Anwendungszubereitung, eingesetzt. Die Gesamtmenge an direktziehenden Farbstoffen beträgt vorzugsweise höchstens 3 Gew.-%.

Besonders gut als Vorstufen naturanaloger Haarfarbstoffe geeignet sind Derivate des 5,6-Dihydroxyindolins sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-di-hydroxyindolin sowie 5,6-Dihydroxyindolin-2-carbonsäure. Besonders hervorzuheben sind N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, insbesondere N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

In einer weiteren, bevorzugten Ausführungsform der vorliegenden Erfindung enthält das Mittel weiterhin mindestens ein amphoteres Tensid. Unter amphoteren Tensiden werden solche oberflächenaktiven Verbindungen verstanden, die ausser einer C₈-C₂₄-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe.

Erfindungsgemäß bevorzugte amphotere Tenside sind Verbindungen gemäß Formel (I) und/oder dessen physiologisch verträglichem Salze, 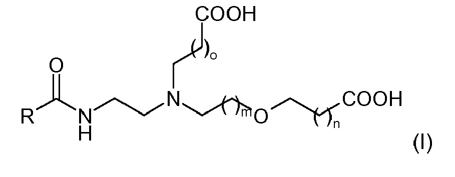 worin R für eine lineare oder verzweigte, gesättigte oder ungesättigte C₁₀-C₂₂-Alkylgruppe steht und m, n und o jeweils unabhängig voneinander für eine ganze Zahl 1, 2 oder 3 steht.

In einer bevorzugten Ausführungsform steht der Rest R gemäß Formel (I) für eine lineare C₁₂-C₁₈-Alkylgruppe. Besonders bevorzugte Verbindungen der Formel (I) werden unter der INCI-Bezeichnung Disodium Cocoamphodipropionate mit den Handelsnamen Miranol^{®} C2M SF conc. (Rhodia), Amphoterge^{®} K-2 (Lonza) und Monateric^{®} CEM-38 (Unichema) vermarktet. Dabei leitet sich die Gruppe R-CO- gemäß Formel (I) von Fettsäuren des Kokosnussöls ab.

Der Zusatz im erfindungsgemäßen Mittel bewirkt eine hervorragende Avivage, insbesondere eine verbesserte Nasskämmbarkeit.

Die amphoteren Tenside sind in dem erfindungsgemäßen Mittel bevorzugt in einer Menge von 0,05 bis 30 Gew.-%, besonders bevorzugt von 0,1 bis 20 Gew.-%, jeweils bezogen auf das Gewicht des Mittels, enthalten.

In einer weiteren, bevorzugten Ausführungsform der vorliegenden Erfindung enthält das Mittel weiterhin mindestens ein kationisches oder amphoteres Polymer.

Die kationischen oder amphoteren Polymere sind in dem erfindungsgemäßen Mittel bevorzugt in einer Menge von 0,01 bis 10 Gew.-%, besonders bevorzugt von 0,05 bis 5 Gew.-%, jeweils bezogen auf das Gesamtgewicht des anwendungsbereiten Mittels, enthalten.

Unter dem Begriff amphotere Polymere werden solche Polymere verstanden,
- die im Molekül sowohl freie Aminogruppen als auch freie -COOH- oder SO₃H-Gruppen enthalten und zur Ausbildung innerer Salze befähigt sind,
- zwitterionische Polymere, die im Molekül quartäre Ammoniumgruppen und -COO⁻-Gruppen oder -SO₃⁻-Gruppen enthalten, sowie
- Polymere, die -COOH-Gruppen oder SO₃H-Gruppen und quartäre Ammoniumgruppen enthalten.

Die oben genannten Polymere mit quartären Ammoniumgruppen werden erfindungsgemäß bevorzugt als amphotere Polymere eingesetzt.

Ein Beispiel für ein erfindungsgemäß einsetzbares Amphopolymer ist das unter der Bezeichnung Amphomer^{®} erhältliche Acrylharz, das ein Copolymeres aus t-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt.

Weitere erfindungsgemäß einsetzbare amphotere Polymere sind die in der britischen Offenlegungsschrift 2 104 091, der europäischen Offenlegungsschrift 47 714, der europäischen Offenlegungsschrift 217 274, der europäischen Offenlegungsschrift 283 817 und der deutschen Offenlegungsschrift 28 17 369 genannten Verbindungen.

Erfindungsgemäß bevorzugte amphotere Polymere sind solche Polymerisate, in denen sich eine kationische Gruppe ableitet von mindestens einem der folgenden Monomere:
(M1) Monomeren mit quartären Ammoniumgruppen der allgemeinen Formel (II), 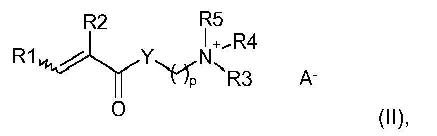 in der R1 und R2 unabhängig voneinander für Wasserstoff oder eine Methylgruppe stehen, R3, R4 und R5 unabhängig voneinander für Alkylgruppen mit 1 bis 4 Kohlenstoff-Atomen stehen, Y eine NH-Gruppe oder ein Sauerstoffatom, n eine ganze Zahl von 2 bis 5 und A⁻ ein physiologisch verträgliches Anion einer organischen oder anorganischen Säure ist,
(M2) Monomeren mit quartären Ammoniumgruppen der allgemeinen Formel (III), 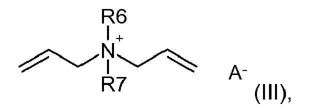 worin R6 und R7 unabhängig voneinander stehen für eine C₁-C₄-Alkylgruppe, insbesondere für eine Methylgruppe und
   A⁻ ein physiologisch verträgliches Anion einer organischen oder anorganischen Säure ist,

Wenn die kationische Gruppe der amphoteren Polymerisate sich vom Monomer des Typs (M1) ableiten, stehen in Formel (II) die Reste R3, R4 und R5 bevorzugt für Methylgruppen, Y ist bevorzugt eine NH-Gruppe und A⁻ bedeutet bevorzugt ein Halogenid-, Methoxysulfat- oder Ethoxysulfat-Ion. Besonders bevorzugt ist es in diesem Falle Acrylamidopropyl-trimethylammoniumchlorid als Monomer (M1) zu verwenden.

Wenn die kationische Gruppe der amphoteren Polymerisate sich vom Monomer des Typs (M2) ableiten, stehen in Formel (III) die Reste R6 und R7 bevorzugt für Methylgruppen und A⁻ steht bevorzugt für ein Halogenidion, insbesondere für Chlorid oder Bromid.

Bevorzugte erfindungsgemäße amphotere Polymere sind Polymere, deren anionische Gruppe sich von mindestens einem Monomeren der Formel (IV) und/oder dessen physiologisch verträglichen Salzen ableitet, 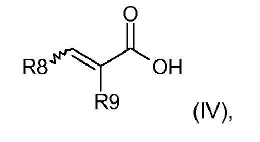 in denen R8 und R9 unabhängig voneinander Wasserstoff oder Methylgruppen sind.

Als Monomeres gemäß Formel (IV) wird für die erfindungsgemäß bevorzugten amphoteren Polymerisate Acrylsäure verwendet.

Besonders bevorzugte Polymere sind Copolymere, die sich von mindestens einem Monomer (M1) bzw. (M2) mit dem Monomer gemäß Formel (IV) ableiten. Insbesondere sind Copolymere bevorzugt, die sich von den Monomeren (M2) und Monomeren gemäß Formel (IV) ableiten. Erfindungsgemäß ganz besonders bevorzugt verwendete amphotere Polymere sind Copolymerisate aus Diallyldimethylammoniumchlorid und Acrylsäure. Diese Copolymerisate werden unter der INCI-Bezeichnung Polyquaternium-22 unter anderem mit dem Handelsnamen Merquat^{®} 280 bzw. Merquat^{®} 281 (Nalco) vertrieben.

Die amphoteren Polymere können sowohl direkt als auch in Salzform, die durch Neutralisation der Polymerisate, beispielsweise mit einem Alkalimetallhydroxid, erhalten wird, erfindungsgemäß eingesetzt werden. Bezüglich der Einzelheiten der Herstellung dieser Polymerisate wird ausdrücklich auf den Inhalt der deutschen Offenlegungsschrift 39 29 973 Bezug genommen.

Das erfindungsgemäß bevorzugte Mengenverhältnis des amphoteren Tensids mit der Formel (I) zum amphoteren oder kationischen Polymer beträgt bevorzugt 1 zu 5 bis 5 zu 1, besonders bevorzugt 1 zu 1 bis 1 zu 2.

Vorzugsweise stellen die anwendungsbereiten Färbemittel fließfähige Zubereitungen dar.

Bevorzugt enthalten die fließfähigen Zubereitungen zusätzlich als oberflächenaktive Substanz ein Emulgator bzw. ein Tensid, wobei oberflächenaktive Substanzen je nach Anwendungsgebiet als Tenside oder als Emulgatoren bezeichnet werden und aus anionischen, kationischen, zwitterionischen und nichtionischen Tensiden und Emulgatoren ausgewählt sind. Diese Stoffe werden nachfolgend ausführlich beschrieben.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie beispielsweise eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein.

Bevorzugte anionische Tenside sind Alkylsulfate, Alkylethersulfate und Ethercarbonsäuren mit 10 bis 18 C-Atomen in der Alkylgruppe und bis zu 12 Glykolethergruppen im Molekül, jeweils in Form der Natrium-, Kalium- und Ammonium- sowie der Mono, Di- und Trialkanolammoniumsalze mit 2 bis 4 C-Atomen in der Alkanolgruppe.

Als zwitterionische Tenside werden solche oberflächenaktiven Verbindungen bezeichnet, die im Molekül mindestens eine quartäre Ammoniumgruppe und mindestens eine Carboxylat-, Sulfonat-oder Sulfat-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Weiterhin hat es sich als vorteilhaft erwiesen, wenn die erfindungsgemäßen Aufhellmittel nichtionogene grenzflächenaktive Stoffe enthalten. Nichtionische Tenside enthalten als hydrophile Gruppe z. B. eine Polyolgruppe, eine Polyalkylenglykolethergruppe oder eine Kombination aus Polyol- und Polyglykolethergruppe.

Als nichtionische Tenside eignen sich insbesondere C₈-C₂₂-Alkylmono- und -oligoglycoside und deren ethoxylierte Analoga. Insbesondere die nichtethoxylierten Verbindungen haben sich als besonders geeignet erwiesen. Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Als weitere bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Besonders bevorzugte nichtionogene oberflächenaktive Substanzen sind dabei wegen der einfachen Verarbeitbarkeit Substanzen, die kommerziell als Feststoffe oder Flüssigkeiten in reiner Form erhältlich sind. Die Definition für Reinheit bezieht sich in diesem Zusammenhang nicht auf chemisch reine Verbindungen. Vielmehr können, insbesondere wenn es sich um Produkte auf natürlicher Basis handelt, Mischungen verschiedener Homologen eingesetzt werden, beispielsweise mit verschiedenen Alkylkettenlängen, wie sie bei Produkten auf Basis natürlicher Fette und Öle erhalten werden. Auch bei alkoxylierten Produkten liegen üblicherweise Mischungen unterschiedlicher Alkoxylierungsgrade vor. Der Begriff Reinheit bezieht sich in diesem Zusammenhang vielmehr auf die Tatsache, dass die gewählten Substanzen bevorzugt frei von Lösungsmitteln, Stellmitteln und anderen Begleitstoffen sein sollen.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder - alkoholate als Katalysatoren verwendet werden. Die Verwendung von Produkten mit eingeengter Homologenverteilung kann bevorzugt sein.

Die anionischen, nichtionischen oder zwitterionischen Tenside werden in Mengen von 0,1 bis 45 Gew.%, bevorzugt 1 bis 30 Gew.% und ganz besonders bevorzugt von 1 bis 15 Gew.%, bezogen auf die Gesamtmenge des anwendungsbereiten Mittels, eingesetzt.

Erfindungsgemäß bevorzugt sind ebenfalls kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf. Weitere erfindungsgemäß verwendbare kationische Tenside stellen die quaternisierten Proteinhydrolysate dar.

Die Alkylamidoamine werden üblicherweise durch Amidierung natürlicher oder synthetischer Fettsäuren und Fettsäureschnitte mit Dialkylaminoaminen hergestellt und zeichnen sich neben einer guten konditionierenden Wirkung speziell durch ihre gute biologische Abbaubarkeit aus. Eine erfindungsgemäß besonders geeignete Verbindung aus dieser Substanzgruppe stellt das unter der Bezeichnung Tegoamid^{®} S 18 im Handel erhältliche Stearamidopropyldimethylamin dar.

Ebenfalls sehr gut biologisch abbaubar sind quaternäre Esterverbindungen, sogenannte "Esterquats". Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis_(2-Palmitoyloxyethyl)dimethylammoniumchlorid, sowie Dehyquart^{®} F-75, Dehyquart^{®} C-4046, Dehyquart^{®} L80 und Dehyquart^{®} AU-35 sind ebenfalls Beispiele für solche Esterquats.

Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

In einer weiteren bevorzugten Ausführungsform kann die Wirkung des erfindungsgemäßen Wirkstoffes durch Emulgatoren gesteigert werden.

Die erfindungsgemäßen Mittel enthalten die Emulgatoren bevorzugt in Mengen von 0,1 bis 25 Gew.-%, insbesondere 0,5 bis 15 Gew.-%, bezogen auf die Gesamtmenge des anwendungsbereiten Mittel.

Bevorzugt können die erfindungsgemäßen Zusammensetzungen mindestens einen nichtionogenen Emulgator mit einem HLB-Wert von 8 bis 18, gemäß den im Römpp-Lexikon Chemie (Hrg. J. Falbe, M. Regitz), 10. Auflage, Georg Thieme Verlag Stuttgart, New York, (1997), Seite 1764, aufgeführten Definitionen enthalten. Nichtionogene Emulgatoren mit einem HLB-Wert von 10-15 können erfindungsgemäß besonders bevorzugt sein.

Unter den genannten Emulgatoren-Typen können die Emulgatoren, welche kein Ethylenoxid und/oder Propylenoxid im Molekül enthalten ganz besonders bevorzugt sein.

Eine oxidative Färbung der Fasern kann in Gegenwart von Oxidationsfarbstoffvorprodukten grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Dieser Aufhelleffekt kann unabhängig von der Färbemethode gewünscht sein. Als Oxidationsmittel kommen Persulfate, Peroxodisulfate, Chlorite, Hypochlorite und insbesondere Wasserstoffperoxid oder dessen Anlagerungsprodukte an Harnstoff, Melamin sowie Natriumborat in Frage.

Bevorzugt wird als Oxidationsmittel Wasserstoffperoxid eingesetzt. Bevorzugt beträgt die Menge an Wasserstoffperoxid im anwendungsbereiten Mittel 0,5 bis 12 Gew.-%, bevorzugt 1 bis 8 Gew.-%, jeweils bezogen auf das anwendungsbereite Mittel.

Solche Oxidationsmittelzubereitungen sind vorzugsweise wässrige, fließfähige Oxidationsmittelzubereitungen. Dabei sind bevorzugte Zubereitungen **dadurch gekennzeichnet, dass** die fließfähige Oxidationsmittelzubereitung - bezogen auf ihr Gewicht - 40 bis 90 Gew.-%, vorzugsweise 50 bis 85 Gew.-%, besonders bevorzugt 55 bis 80 Gew.-%, weiter bevorzugt 60 bis 77,5 Gew.-% und insbesondere 65 bis 75 Gew.-% Wasser enthält.

Erfindungsgemäß kann aber das Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z. B. bestimmte Enzyme, Iodide, Chinone oder Metallionen.

Geeignete Enzyme sind z. B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, oder in situ geringe Mengen Wasserstoffperoxid erzeugen und auf diese Weise die Oxidation der Farbstoffvorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, z. B. Pyranose-Oxidase (mit D-Glucose oder Galactose), Glucose-Oxidase (mit D-Glucose), Glycerin-Oxidase (mit Glycerin), Pyruvat-Oxidase (mit Benztraubensäure oder deren Salzen), Alkohol-Oxidase (mit Alkohol wie MeOH, EtOH), Lactat-Oxidase (mit Milchsäure und deren Salzen), Tyrosinase-Oxidase (mit Tyrosin), Uricase (mit Harnsäure oder deren Salzen), Cholinoxidase (mit Cholin), Aminosäure-Oxidase (mit Aminosäuren).

Die erfindungsgemäßen Mittel können zusätzlich auch Blondier- und/oder Bleichmittel enthalten und somit als Mittel bereitgestellt werden, die gleichzeitig färbend und aufhellend wirken. Solche Mittel werden nachfolgend als "Färbemittel", als "aufhellende Färbemittel" oder als "Färbe- und Aufhellmittel" bezeichnet.

Für die starke Aufhellung sehr dunklen Haares ist der alleinige Einsatz von Wasserstoffperoxid oder dessen Anlagerungsprodukten an organische beziehungsweise anorganische Verbindungen oftmals nicht ausreichend. In diesen Fällen wird in der Regel eine Kombination aus Wasserstoffperoxid und Persulfaten bzw. Peroxodisulfaten eingesetzt, woraus eine Steigerung des Aufhellvermögens der Mittel resultiert.

Daher kann es, sollte der Verbraucher den Wunsch nach einer sehr starken Blondierung verspüren, in einer weiteren Ausführungsform bevorzugt sein, wenn das erfindungsgemäße Färbemittel zusätzlich mindestens ein anorganisches Persulfatsalz bzw. Peroxodisulfatsalz in dem Mittel zum Aufhellen der keratinischen Fasern enthält. Bevorzugte Peroxodisulfatsalze sind Ammoniumperoxodisulfat, Kaliumperoxodisulfat und Natriumperoxodisulfat. Die Peroxodisulfatsalze können in einer Menge von 0,1 bis 25 Gew.-%, insbesondere in einer Menge von 0,5 bis 15 Gew.-%, bezogen auf das Gesamtgewicht des anwendungsbereiten Mittels, enthalten sein. Der Einsatz von Persulfatsalzen bzw. Peroxodisulfatsalzen erfolgt in der Regel in Form eines gegebenenfalls entstaubten Pulvers oder eines in Form gepressten Formkörpers.

Bei einer Anwendung von zusätzlichen Oxidationsmitteln wird das eigentliche Färbe- und/oder Aufhellmittel zweckmäßigerweise unmittelbar vor der Anwendung durch Mischung einer erfindungsgemäßen Zubereitung, enthaltend in einem kosmetischen Träger mindestens ein Oxidationsfarbstoffvorprodukt, mindestens eine basische Aminosäure, 0,3 bis 10 Gew.-% Ammoniumsulfat, bezogen auf das Gesamtgewicht der Anwendungsmischung, und mindestens ein Alkanolamin, sowie einer Zubereitung, enthaltend das zusätzliche Oxidationsmittel, insbesondere Wasserstoffperoxid, hergestellt.

Wird eine starke Aufhellung gewünscht, so ist es erfindungsgemäß bevorzugt, wenn zusätzlich eine Blondierzubereitung, enthaltend mindestens ein anorganisches Persulfatsalz bzw. Peroxodisulfatsalz, der Oxidationsmittelzubereitung vor Vermischung mit der erfindungsgemäßen Färbezubereitung beigemischt wird.

Ein Einsatz bestimmter Metallionen oder -komplexe kann ebenfalls bevorzugt sein, um intensive Färbungen zu erhalten. Geeignete Metallionen sind beispielsweise Zn²⁺, Cu²⁺, Fe²⁺, Fe³⁺, Mn²⁺, Mn⁴⁺, Li⁺, Mg²⁺, Ca²⁺, Ce⁴⁺, V³⁺, Co²⁺, Ru³⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate, Citrate und Tartrate. Durch Verwendung dieser Metallsalze kann sowohl die Ausbildung der Färbung beschleunigt als auch die Farbnuance gezielt beeinflusst werden.

Besonders bevorzugte Mittel 0,0001 bis 2,5 Gew.-%, vorzugsweise 0,001 bis 1 Gew.-% mindestens einer Verbindung aus der Gruppe Kupferchlorid (CuCl₂), Kupfersulfat (CuSO₄), Eisen(II)sulfat, Mangan(II)sulfat, Mangan(II)chlorid, Kobalt(II)chlorid, Cersulfat, Cerchlorid, Vanadiumsulfat, Mangandioxid (MnO₂) enthalten.

Erfindungsgemäß bevorzugt ist auch der Einsatz von sogenannten Komplexbildnern. Komplexbilder sind Stoffe, die Metallionen komplexieren können. Bevorzugte Komplexbildner sind sogenannte Chelatkomplexbildner, also Stoffe, die mit Metallionen cyclische Verbindungen bilden, wobei ein einzelner Ligand mehr als eine Koordinationsstelle an einem Zentralatom besetzt, d. h. mindestens "zweizähnig" ist. Die Zahl der gebundenen Liganden hängt von der Koordinationszahl des zentralen Ions ab.

Gebräuchliche und im Rahmen der vorliegenden Erfindung bevorzugte Chelatkomplexbildner sind beispielsweise Polyoxycarbonsäuren, Polyamine, Ethylendiamintetraessigsäure (EDTA), Nitrilotriessigsäure (NTA) und Hydroxyethandiphosphonsäuren bzw. deren Alkalisalze. Auch komplexbildende Polymere, also Polymere, die entweder in der Hauptkette selbst oder seitenständig zu dieser funktionelle Gruppen tragen, die als Liganden wirken können und mit geeigneten MetallAtomen in der Regel unter Bildung von Chelat-Komplexen reagieren, sind erfindungsgemäß einsetzbar. Die Polymer-gebundenen Liganden der entstehenden Metall-Komplexe können dabei aus nur einem Makromolekül stammen oder aber zu verschiedenen Polymerketten gehören. Letzteres führt zur Vernetzung des Materials, sofern die komplexbildenden Polymere nicht bereits zuvor über kovalente Bindungen vernetzt waren.

Eine weitere Substanzklasse mit komplexbildenden Eigenschaften stellen die Phosphonate dar. Dabei handelt es sich insbesondere um Hydroxyalkan- bzw. Aminoalkanphosphonate. Unter den Hydroxyalkanphosphonaten ist das 1-Hydroxyethan-1,1-diphosphonat (HEDP) von besonderer Bedeutung. Es wird vorzugsweise als Natriumsalz eingesetzt, wobei das Dinatriumsalz neutral und das Tetranatriumsalz alkalisch (pH 9) reagiert. Als Aminoalkanphosphonate kommen vorzugsweise Ethylendiamintetramethylenphosphonat (EDTMP), Diethylentriaminpentamethylenphosphonat (DTPMP) sowie deren höhere Homologe in Frage. Sie werden vorzugsweise in Form der neutral reagierenden Natriumsalze, z. B. als Hexanatriumsalz der EDTMP bzw. als Hepta-und Octa-Natriumsalz der DTPMP, eingesetzt. Als Komplexbildner wird dabei aus der Klasse der Phosphonate bevorzugt HEDP verwendet. Die Aminoalkanphosphonate besitzen zudem ein ausgeprägtes Schwermetallbindevermögen. Dementsprechend kann es, insbesondere wenn die Mittel auch Bleiche enthalten, bevorzugt sein, Aminoalkanphosphonate, insbesondere DTPMP, einzusetzen, oder Mischungen aus den genannten Phosphonaten zu verwenden. Diese Stoffe werden nachstehend beschrieben.

Erfindungsgemäß bevorzugte Komplexbildner sind Phosphonate, vorzugsweise Hydroxyalkan- bzw. Aminoalkanphosphonate und insbesondere 1-Hydroxyethan-1,1-diphosphonat (HEDP) bzw. dessen Di- oder Tetranatriumsalz und/oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz.

Insbesondere bevorzugt sind in den erfindungsgemäßen Mitteln zusätzlich Proteine und/ oder Proteinderivate pflanzlicher oder tierischer Herkunft wie beispielsweise Erbsen-, Soja-, Weizen-und Mandelproteinhydrolysat oder Akazienprotein sowie Collagen- oder Keratinhydrolysat enthalten. Die erfindungsgemäßen Mittel enthalten die Proteine und/oder Proteinderivate pflanzlicher oder tierischer Herkunft bevorzugt in einer Menge von 0,1 bis 10 Gew.-%, insbesondere 0,5 bis 3 Gew.-%, jeweils bezogen auf das Gewicht des gesamten Mittels.

Ferner können die erfindungsgemäßen Mittel weitere Wirk-, Hilfs- und Zusatzstoffe enthalten, wie beispielsweise nichtionische Polymere, kationische Polymere, zwitterionische und amphotere Polymere, anionische Polymere, Verdickungsmittel, Strukturanten wie Glucose, Maleinsäure und Milchsäure, haarkonditionierende Verbindungen wie Phospholipide sowie Silikonöle, Parfümöle, Dimethylisosorbid und Cyclodextrine, faserstrukturverbessernde Wirkstoffe, insbesondere Mono-, Di- und Oligosaccharide wie beispielsweise Glucose, Galactose, Fructose, Fruchtzucker und Lactose, Entschäumer wie Silikone, bevorzugt Dimethicon, Farbstoffe zum Anfärben des Mittels, Antischuppenwirkstoffe wie Piroctone Olamine, Zink Omadine und Climbazol, Lichtschutzmittel, insbesondere Derivate von Benzophenon, Zimtsäure und Triazin, Konsistenzgeber wie Zuckerester, Polyolester oder Polyolalkylether, Fette und Wachse, Fettsäurealkanolamide, Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Trübungsmittel wie Latex, Styrol/PVP- und Styrol/Acrylamid-Copolymere, Perlglanzmittel wie Ethylenglykolmono- und -distearat sowie PEG-3-distearat, Pigmente, Stabilisierungsmittel für Wasserstoffperoxid und andere Oxidationsmittel, Treibmittel wie Propan-Butan-Gemische, N₂O, Dimethylether, CO₂ und Luft, und Antioxidantien.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Um die Keratinfasern und auch die Haut so weit wie möglich zu schonen, ist die Einstellung eines zu hohen pH-Wertes jedoch nicht wünschenswert. Daher ist es bevorzugt, wenn das gebrauchsfertige Haarfärbepräparat bevorzugt einen pH-Wert im Bereich von 6 bis 12 aufweist. Besonders bevorzugt ist die Anwendung der Haarfärbemittel in einem schwach alkalischen Milieu, bevorzugt bei einem pH-Wert im Bereich von 8,0 bis 9,5. Zur Einstellung des pH-Werts sind dem Fachmann gängige Acidifizierungs- und Alkalisierungsmittel geläufig. Die zur Einstellung des bevorzugten pH-Wertes erfindungsgemäß verwendbaren Alkalisierungsmittel können aus der Gruppe, die gebildet wird aus Alkalimetallhydroxiden, Alkalimetallcarbonaten Alkalimetallmetasilikaten, Alkalimetallphosphaten und Alkalimetallhydrogenphosphaten ausgewählt werden. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren.

Bei den pH-Werten im Sinne der vorliegenden Erfindung handelt es sich um pH-Werte, die bei einer Temperatur von 22 °C gemessen wurden.

Ein zweiter Gegenstand der vorliegenden Erfindung ist ein Verfahren zum Färben menschlicher Haare, bei dem ein erfindungsgemäßes Mittel gemäß obiger Vorgaben auf das Haar aufgetragen wird, für eine Einwirkzeit von 2 bis 20 Minuten, bevorzugt von 5 bis 15 Minuten auf dem Haar belassen wird, und anschließend das Haar ausgespült wird.

Eine weitere bevorzugte Ausführungsform des erfindungsgemäßen Verfahrens zum Färben und gegebenenfalls Aufhellen von menschlichen Haaren liegt vor, wenn eine Zusammensetzung in einem kosmetischen Träger, enthaltend mindestens ein Oxidationsfarbstoffvorprodukt, mindestens eine basische Aminosäure, Ammoniumsulfat zu 0,3 bis 10 Gew.-%, bezogen auf das Gesamtgewicht der Anwendungsmischung, und mindestens ein Alkanolamin, mit einer Oxidationsmittelzubereitung, enthaltend Wasserstoffperoxid, zu einer homogenen Zusammensetzung vermischt, diese auf das Haar aufgebracht wird, für eine Einwirkzeit von 2 bis 30 Minuten, bevorzugt von 5 bis 15 Minuten auf dem Haar belassen wird, und anschließend das Haar ausgespült wird.

Die im Vergleich zu Handelsprodukten kurze Einwirkzeit im erfindungsgemäßen Verfahren garantiert bei gleichbleibenden Färbeergebnis ebenfalls eine verringerte Schädigung der Haare durch die verkürzte Kontaktzeit von schädigenden Substanzen mit dem Haar.

Bezüglich weiterer bevorzugter Ausführungsformen der erfindungsgemäßen Verfahren gilt mutatis mutandis das zu den erfindungsgemäßen Mitteln Gesagte.

Die Anwendungstemperaturen beim erfindungsgemäßen Färbeverfahren können in einem Bereich zwischen 15 und 45 °C liegen. Nach einer Einwirkungszeit wird das Haarfärbemittel durch Ausspülen von dem zu färbenden Haar entfernt. Das Nachwaschen mit einem Shampoo kann entfallen, wenn ein stark tensidhaltiger Träger, z. B. ein Färbeshampoo, verwendet wurde.

Wie bereits erwähnt, können die erfindungsgemäßen Mittel auch direkt vor der Anwendung aus zwei oder mehreren getrennt verpackten Zubereitungen hergestellt werden. Dies bietet sich insbesondere zur Trennung inkompatibler Inhaltsstoffe an, um eine vorzeitige Reaktion zu vermeiden. Eine Auftrennung in Mehrkomponentensysteme bietet sich insbesondere dort an, wo Inkompatibilitäten der Inhaltsstoffe zu erwarten oder zu befürchten sind. Das anwendungsbereite Mittel wird bei solchen Systemen vom Verbraucher direkt vor der Anwendung durch Vermischen der Komponenten hergestellt. Ein Färbe- und/oder Aufhellungsmittel, bei dem die Oxidationsfarbstoffvorprodukte zunächst getrennt von der Oxidationsmittelzubereitung, enthaltend bevorzugt Wasserstoffperoxid, vorliegen, ist dabei bevorzugt.

Ein dritter Gegenstand der vorliegenden Erfindung ist daher eine Mehrkomponentenverpackungseinheit (Kit-of-Parts), enthaltend mindestens zwei getrennt konfektionierte Container, wobei ein Container eine Zusammensetzung in einem kosmetischen Träger, enthaltend mindestens ein Oxidationsfarbstoffvorprodukt, mindestens eine basische Aminosäure, Ammoniumsulfat zu 0,3 bis 10 Gew.-%, bezogen auf das Gesamtgewicht der Anwendungsmischung, und mindestens ein Alkanolamin, enthält und ein Container eine Oxidationsmittelzusammensetzung, enthaltend mindestens ein chemisches Oxidationsmittel, insbesondere Wasserstoffperoxid, enthält.

Wird eine besonders starke Aufhellwirkung durch Einsatz von Persulfatsalzen bzw. Peroxodisulfatsalzen gewünscht, ist es erfindungsgemäß bevorzugt, diese der erfindungsgemäßen Mehrkomponentenverpackungseinheit (Kit-of-Parts) in Form eines gegebenenfalls entstaubten Pulvers oder eines in Form gepressten Formkörpers als separat verpackte, zusätzliche Komponente beizufügen.

Bevorzugt enthält die Mehrkomponentenverpackungseinheit (Kit-of-Parts) zusätzlich eine Gebrauchsleitung. Darüber hinaus kann es bevorzugt sein, wenn weiterhin eine Applikationshilfe, wie beispielsweise ein Kamm oder ein Pinsel, und/oder eine persönliche Schutzausrüstung, wie beispielsweise Einweg-Handschuhe dem Kit beigefügt ist.

Bezüglich weiterer bevorzugter Ausführungsformen der Mehrkomponentenverpackungseinheit (Kit-of-Parts) gilt mutatis mutandis das zu den erfindungsgemäßen Mitteln Gesagte.

Ein weiterer Gegenstand der Erfindung ist die Verwendung der Mittel des ersten Erfindungsgegenstandes zum Färben menschlicher Haare bei gleichzeitiger Pflege der Haare.

Ein weiterer Gegenstand der Erfindung ist die Verwendung der Mittel des ersten Erfindungsgegenstandes zum Färben menschlicher Haare bei gleichzeitigem Schutz vor Schädigungen der Haare.

Ein weiterer Gegenstand der Erfindung ist schließlich die Verwendung der Mittel des ersten Erfindungsgegenstandes zum Färben menschlicher Haare bei verringerten Schädigungen der Haare.

Bezüglich weiterer bevorzugter Ausführungsformen der erfindungsgemäßen Verwendungen gilt mutatis mutandis das zu den erfindungsgemäßen Mitteln Gesagte.

Die nachfolgenden Beispiele sollen bevorzugte Ausführungsformen der Erfindung erläutern, ohne sie jedoch einzuschränken.

### Beispiele

### 1. Haarschädigung

### 1.1. Herstellung einer Färbecreme

**Aus den aufgelisteten Bestandteilen (Angaben in Gew.-%) wurden Färbecremes wie folgt hergestellt:**

| | **E1** | **V1** | **V2** |
|---|---|---|---|
| Hydrenol D | 6,0 | 6,0 | 6,0 |
| Lorol techn. | 3,6 | 3,6 | 3,6 |
| Eumulgin B2 | 0,3 | 0,3 | 0,3 |
| Eumulgin B3 | 0,3 | 0,3 | 0,3 |
| Mergital CS 50 A | 1,2 | 1,2 | 1,2 |
| 1,2-Propandiol | 0,6 | 0,6 | 0,6 |
| Xanthan | 0,12 | 0,12 | 0,12 |
| Merquat 281 | 3,0 | 3,0 | 3,0 |
| Amphoterge K-2 | 2,0 | 2,0 | 2,0 |
| Crodafos CES | 2,0 | 2,0 | 2,0 |
| Arginin | 1,0 | - | - |
| Ammoniumsulfat, techn. rein | 2,0 | 2,0 | 2,0 |
| Monoethanolamin | 3,7 | 3,7 | 4,04 |
| p-Toluylendiaminsulfat | 1,52 | 1,52 | 1,52 |
| 2-Methylresorcin | 0,47 | 0,47 | 0,47 |
| m-Aminophenol | 0,16 | 0,16 | 0,16 |
| 4-Chlorresorcin | 0,17 | 0,17 | 0,17 |
| Resorcin | 0,13 | 0,13 | 0,13 |
| 2-Amino-6-chloro-4-nitrophenol | 0,03 | 0,03 | 0,03 |
| Ascorbinsäure | 0,2 | 0,2 | 0,2 |
| Natriumsulfit, wasserfrei, 96% | 0,2 | 0,2 | 0,2 |
| HEDP, 60% | 0,2 | 0,2 | 0,2 |
| Natriumsilikat 40 / 42 | 0,5 | 0,5 | 0,5 |
| Wasser | ad 100 | ad 100 | ad 100 |

- Hydrenol^{®} D: C₁₆-C₁₈-Fettalkohol (INCI-Bezeichnung: Cetearyl alcohol) (Cognis)
- Lorol^{®} tech.: C₁₂-C₁₈-Fettalkohol (INCI-Bezeichnung: Coconut alcohol) (Cognis)
- Eumulgin^{®} B2: C₁₆-C₁₈-Fettalkohol, ethoxyliert (20 EO) (INCI-Bezeichnung: Ceteareth-20) (Cognis)
- Eumulgin^{®} B3: C₁₆-C₁₈-Fettalkohol, ethoxyliert (30 EO) (INCI-Bezeichnung: Ceteareth-30) (Cognis)
- Mergital^{®} CS 50: C₁₆-C₁₈-Fettalkohol, ethoxyliert (50 EO) (INCI-Bezeichnung: Ceteareth-50) (Cognis)
- Merquat^{®} 281: Dimethyldiallylammoniumchlorid/Acrylsäure Copolymer (INCI-Bezeichnung: Polyquaternium-22) (Nalco)
- Amphoterge^{®} K-2: (INCI-Bezeichnung: Disodium Cocoamphodipropionate) (Lonza)
- Crodafos^{®} CES: (INCI-Bezeichnung: Cetearyl alcohol, Dicetyl phosphate, Ceteth-10 phosphate) (Croda Oleochemicals)
- Natriumsilikat 40/42: Natronwasserglas
Hydrenol D, Lorol, Eumulgin B1, Eumulgin B2, Mergital CS 50 und Xanthan FN wurden zusammen bei 80 °C aufgeschmolzen und mit einem Teil der Wassermenge dispergiert. Anschließend wurden die restlichen Rezepturbestandteile unter Rühren der Reihe nach eingearbeitet. Dann wurde mit Wasser auf 100 Gew.-% aufgefüllt und die Formulierung kalt gerührt.

Die Rezeptur E1 ist ein erfindungsgemäßes Beispiel. Bei den Rezepturen V1 und V2 handelt es sich um nicht erfindungsgemäße Vergleichsrezepturen ohne basische Aminosäure, wobei V1 die gleiche Menge Monoethanolamin wie E1 enthält, während V2 mehr Monoethanolamin enthält, so dass die identische Alkalisierungsmittelmenge verglichen mit E1 erreicht wird.

### 1.2. Vermischen mit der Entwicklerdispersion und Applikation

Jede Färbecreme wurde im Gewichtsverhältnis 1:1 mit einer wie folgt zusammengesetzten Entwicklerdispersion ausgemischt. Der Mischungs-pH-Wert betrug jeweils 9.3.

| **Rohstoff** | **Gew.-%** |
|---|---|
| Natronlauge, 45 Gew.-%, techn. | 0,82 |
| Dipicolinsäure | 0,10 |
| Dinatriumpyrophosphat | 0,03 |
| HEDP, 60% | 1,50 |
| Texapon NSO | 2,00 |
| Dow Corning DB 110 A | 0,07 |
| Aculyn 33A | 4,50 |
| Wasserstoffperoxid 50 % | 10,00 |
| Wasser | ad 100 |

- Texapon^{®} NSO: Laurylethersulfat, Natriumsalz (ca. 27,5% Aktivsubstanz; INCI-Bezeichnung: Sodium Laureth Sulfate) (Cognis)
- Aculyn^{®} 33: Acrylpolymer (ca. 28% Festkörper in Wasser; INCI-Bezeichnung: Acrylates Copolymer) (Rohm & Haas)
- Dow Corning^{®}DB 110 A: nicht ionische Silikonemulsion (INCI-Bezeichnung: Dimethicon) (Dow Corning)

Für den Färbung wurde auf Strähnen (Codes: Kerling ENH 10/0 bzw. Kerling ENH 6/0) von ca. 0,7 g Gewicht jeweils die 4-fache Menge der fertigen Anwendungsmischung appliziert. Nachdem die Strähnen für 10 Minuten bei 32 °C gefärbt wurden, wurden sie mit einem handelsüblichen Shampoo gewaschen und mit einem Föhn getrocknet.

Die Ausfärbung ergab für E1 eine warme, dunkelbraune Färbung, die deutliche Vorteile bezüglich ihrer Waschbeständigkeit (20fache Wiederholung) und Grauabdeckung gegenüber den Ausfärbungen der Vergleichsrezepturen V1 und V2.

### 1.3. Auswertung der Haarschädigung

### 1.3.1 Methodenbeschreibung zur NIR-Analytik von behandeltem Haar

### Qualitative Bestimmung der Haarstrukturschädigung mittels Clusteranalyse

Haare können durch oxidative Behandlung wie Färben oder Dauerwellen geschädigt werden.

Cystin ist ein Bestandteil des menschlichen Haares. Cystin zeigt bei Spektrum bei einer Wellenlänge von 6200 cm⁻¹ - 5500 cm⁻¹ typische Schwingungen im NIR (Nah-Infra-Rot). Die in Cystin enthaltene Disulfid-Brücke wird bei einer Haarbehandlung oxidativ gebrochen, und der resultierende Thiol durch weitere Oxidation in die Sulfonsäure-Gruppe der Cysteinsäure überführt. Gleichzeitig verändert sich das Haar hinsichtlich des Cysteinsäuregehalts, welcher mit stärker werdender, oxidativer Schädigung zunimmt. Cysteinsäure zeigt sich im NIR-Spektrum bei einer Wellenlänge von 5020 cm⁻¹ - 4020 cm⁻¹. Die Abnahme an Cystin und die Zunahme an Cysteinsäure stellt daher einen Indikator für die Schädigung des Haares dar.

Die Aufnahme der Spektren wird mit einem MPATM FT-NIR-Spektrometer der Bruker Optik GmbH (Ettlingen, BRD) durchgeführt. Der Nah-Infrarot (NIR)-Bereich umfasst den Wellenzahlbereich von 12500 cm⁻¹ bis 4000 cm⁻¹ und ist für Oberton- und Kombinationsschwingungen von z.B. CH-, OH- und NH-Gruppen charakteristisch. Die Messung der Proben erfolgt mit dem Integrationskugelmodul an sechs verschiedenen Probenpositionen in diffuser Reflexion. Für die Analyse der gemessenen NIR-Spektren wird der Wellenzahlbereich 8000 cm⁻¹ - 4020 cm⁻¹ gewählt. Für die chemometrische Auswertung wird das geräteunabhängige Softwarepaket Unscrambler (Fa. CAMO, Oslo, Norwegen) verwendet.

Anhand einer PCA (Principal Compound Analysis) kann man die Separation der Spektren in sogenannte "Cluster" erkennen Je größer die Abstände der Cluster zu dem der unbehandelten Muster sind, desto größer ist die spektrale Abweichung einhergehend mit dem Ausmaß der Haarstrukturveränderung. Auf diese Art und Weise lassen sich die Haare nach Behandlung mit Aufhellern und anschließender Vermessung im NIR in Cluster einteilen. Die Stärke der Veränderung des behandelten Haares ist sofort erkennbar.

### Quantitative Bestimmung der Haarstrukturschädigung mittels Kalibrationserstellung

NIR-Spektren lassen sich auch quantitativ auswerten, wenn Referenzwerte vorhanden sind. Bei der Modellbildung wird mit Hilfe eines Partial Least Squares (PLS)-Algorithmus eine Korrelation zwischen spektroskopischen Daten und den entsprechenden Konzentrationswerten der einzelnen Komponenten berechnet. Referenzwerte erhält man, indem man Proben im Labor über ein anderes unabhängiges analytisches Verfahren quantitativ bestimmt. Das PLS-Modell wird durch Veränderung des Frequenzbereiches, mathematische Datenvorbehandlung, Auswahl der geeigneten Faktorenzahl und automatischer Ausreißererkennung verbessert.

Eine detaillierte Beschreibung der Methode findet sich in Evaluation of Physical Properties of Human Hair by Diffuse Reflectance Near-Infrared Spectroscopy, Y. Miyamae, Y. Yamakawa und Y. Ozaki in Applied Spectroscopy, 2007, Vol. 61 (2), S. 212 ff.

### 1.3.2 Ergebnisse

| Haarprobe | | NIR-Analsyenwert Cysteinsäure [mol/100 mol Aminosäure] |
|---|---|---|
| Kerling 10/0 | unbehandelt | 3,8 |
| | gefärbt mit E1 | 4,1 |
| | gefärbt mit V1 | 4,3 |
| | gefärbt mit V2 | 4,5 |
| Kerling 6/0 | unbehandelt | 0,9 |
| | gefärbt mit E1 | 1,2 |
| | gefärbt mit V1 | 1,3 |
| | gefärbt mit V2 | 1,4 |

Die Cysteinsäuregehalte der Haarproben gemäß 1.2 wurden mittels NIR-Messungen bestimmt. Die Ergebnisse zeigen für beide Haartypen geringere Mengen an Cysteinsäure für das erfindungsgemäße Färbemittel E1 verglichen mit den Vergleichsfärbemitteln. Dadurch wird die signifikant geringere Haarschädigung durch die erfindungsgemäße Färbezusammensetzung verdeutlicht.

### 2. Weitere erfindungsgemäße Färbecremes:

| | **E2** | **E3** | **E4** |
|---|---|---|---|
| Hydrenol D | 6,0 | 6,0 | 6,0 |
| Lorol techn. | 3,6 | 3,6 | 3,6 |
| Eumulgin B1 | 0,3 | 0,3 | 0,3 |
| Eumulgin B2 | 0,3 | 0,3 | 0,3 |
| Mergital CS 50 A | 1,2 | 1,2 | 1,2 |
| 1,2-Propandiol | 0,6 | 0,6 | 0,6 |
| Xanthan FN | 0,12 | 0,12 | 0,12 |
| Merquat 281 | 3,0 | 3,0 | 3,0 |
| Amphoterge K-2 | 2,0 | 2,0 | 2,0 |
| Crodafos CES | 2,0 | 2,0 | 2,0 |
| Arginin | 1,0 | 1,0 | 1,0 |
| Ammoniumsulfat, techn. rein | 2,0 | 2,0 | 2,0 |
| Monoethanolamin | 5,4 | 3,45 | 3,45 |
| p-Toluylendiamin sulfat | 2,65 | 1,00 | 0,39 |
| 1-Hydroxyethyl-4,5-diamino-pyrazol sulfat | - | 0,25 | - |
| 4-Amino-3-methylphenol | - | 0,40 | 0,84 |
| 2-Methylresorcin | 0,11 | 0,20 | - |
| m-Aminophenol | 0,30 | 0,10 | 0,23 |
| p-Amino-o-Cresol | - | 0,15 | 0,29 |
| 2-Amino-3-hydroxypyridin | - | 0,45 | 0,17 |
| Resorcin | 0,90 | 0,90 | 0,43 |
| 1-Naphthol | - | - | 0,06 |
| 2-Amino-4-hydroxyethyl-amino-anisol sulfat | 0,03 | - | - |
| 4-Amino-3-nitrophenol | - | - | 0,06 |
| 2-Amino-6-chloro-4-nitrophenol | - | - | 0,23 |
| Ascorbinsäure | 0,05 | 0,05 | 0,2 |
| Natriumsulfit, wasserfrei, 96% | 0,2 | 0,2 | 0,2 |
| HEDP, 60% | 0,2 | 0,2 | 0,2 |
| Natriumsilikat 40 / 42 | 0,5 | 0,5 | 0,5 |
| Wasser | ad 100 | ad 100 | ad 100 |

Die Ausfärbungen wurden analog zu Beispiel 2.1 durchgeführt und ergaben für E2 eine warme, dunkelbraune Färbung, für E3 eine rötlichbraune Färbung und für E4 ein milchschokoladenfarbige Färbung.

## Patentansprüche

1. Mittel zum Färben keratinischer Fasern, insbesondere menschlicher Haare, enthaltend in einem kosmetischen Träger mindestens ein Oxidationsfarbstoffvorprodukt, **dadurch gekennzeichnet, dass** das Färbemittel zusätzlich
a) mindestens eine basische Aminosäure,
b) 0,3 bis 10 Gew.-% Ammoniumsulfat, bezogen auf das Gesamtgewicht der Anwendungsmischung,
c) und mindestens ein Alkanolamin enthält.

2. Mittel nach Anspruch 1, **dadurch gekennzeichnet, dass** die basische Aminosäure Arginin ist.

3. Mittel nach einem der Ansprüche 1 oder 2, **dadurch gekennzeichnet, dass** Ammoniumsulfat zu 0,5 bis 5 Gew.-%, insbesondere zu 0,8 bis 3 Gew.-% und ganz besonders bevorzugt zu 0,8 bis 2 Gew.-%, jeweils bezogen auf das Gesamtgewicht der Anwendungsmischung, enthalten ist.

4. Mittel nach einem der Ansprüche 1 bis 3, **dadurch gekennzeichnet, dass** das Mittel ammoniakarm, bevorzugt ammoniakfrei ist.

5. Mittel nach einem der Ansprüche 1 bis 4, **dadurch gekennzeichnet, dass** das Alkanolamin ausgewählt aus der Gruppe, gebildet aus Monoethanolamin, Monoisopropanolamin, 2-Amino-2-methylpropanol, 2-Amino-2-methyl-1,3-propandiol, 2-Amino-2-ethyl-1,3-propandiol, 2-Amino-2-methylbutanol, 2-Aminobutanol, N,N-Dimethyl-ethanolamin, Methylglucamin, Triethanolamin, Diethanolamin und Triisopropanolamin.

6. Mittel nach einem der Ansprüche 1 bis 5, **dadurch gekennzeichnet, dass** das Alkanolamin Monoethanolamin ist.

7. Mittel nach einem der Ansprüche 1 bis 6, **dadurch gekennzeichnet, dass** weiterhin mindestens ein amphoteres Tensid enthalten ist.

8. Mittel nach Anspruch 7, **dadurch gekennzeichnet, dass** als amphoteres Tensid ein Tensid gemäß Formel (I) und/oder dessen physiologisch verträgliches Salz, 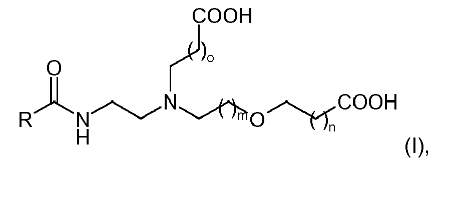 worin R für eine lineare oder verzweigte, gesättigte oder ungesättigte C₁₀ bis C₂₂-Alkylgruppe steht und m, n und o jeweils unabhängig voneinander für eine ganze Zahl 1, 2 oder 3 steht,
enthalten ist.

9. Mittel nach einem der Ansprüche 1 bis 8, **dadurch gekennzeichnet, dass** es zusätzlich ein kationisches oder amphoteres Polymer enthält.

10. Mittel nach Anspruch 9, **dadurch gekennzeichnet, dass** das amphotere Polymer ein Copolymer ist, welches sich ableitet von mindestens einem kationischen Monomer, ausgewählt aus
(M1) Monomeren mit quartären Ammoniumgruppen der allgemeinen Formel (II), 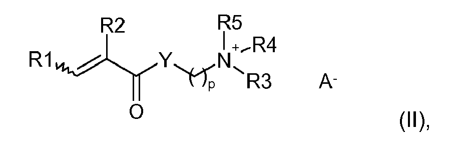 in der R1 und R2 unabhängig voneinander für Wasserstoff oder eine Methylgruppe stehen, R3, R4 und R5 unabhängig voneinander für Alkylgruppen mit 1 bis 4 Kohlenstoff-Atomen stehen, Y eine NH-Gruppe oder ein Sauerstoffatom, n eine ganze Zahl von 2 bis 5 und A⁻ ein physiologisch verträgliches Anion einer organischen oder anorganischen Säure ist,
(M2) Monomeren mit quartären Ammoniumgruppen der allgemeinen Formel (III),
i. 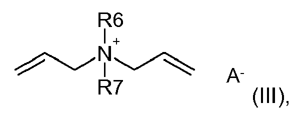 worin R6 und R7 unabhängig voneinander stehen für eine C₁ bis C₄-Alkylgruppe, insbesondere für eine Methylgruppe und
A⁻ ein physiologisch verträgliches Anion einer organischen oder anorganischen Säure ist,
und von mindestens einem anionischen Monomer der Formel (IV) und/oder dessen physiologisch verträglichen Salzen,
ii. 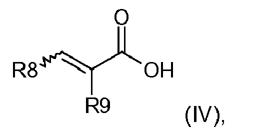 in denen R8 und R9 unabhängig voneinander Wasserstoff oder Methylgruppen sind.

11. Mittel nach einem der Ansprüche 9 bis 10, **dadurch gekennzeichnet, dass** das amphotere Polymer ausgewählt wird aus Copolymeren, abgeleitet von mindestens einem Monomeren (M2) und von mindestens einem Monomeren gemäß Formel (IV).

12. Mittel nach einem der Ansprüche 1 bis 11, **dadurch gekennzeichnet, dass** zusätzlich mindestens ein direktziehender Farbstoff enthalten ist.

13. Verfahren zum Färben menschlicher Haare, bei dem ein Mittel nach einem der Ansprüche 1 bis 12 auf das Haar aufgetragen wird, für eine Einwirkzeit von 2 bis 20 Minuten, bevorzugt von 5 bis 15 Minuten auf dem Haar belassen wird, und anschließend das Haar ausgespült wird.

14. Kit-of-Parts, enthaltend mindestens zwei getrennt konfektionierte Container, wobei ein Container eine Oxidationsfärbemischung gemäß einem Ansprüche 1 bis 12 enthält und ein Container eine Oxidationsmittelzusammensetzung, enthaltend mindestens ein chemisches Oxidationsmittel, insbesondere Wasserstoffperoxid, enthält.

15. Verwendung eines Mittels gemäß einem der Ansprüche 1 bis 12 zum Färben menschlicher Haare bei gleichzeitiger Pflege der Haare.
